# EUROPÄISCHE PATENTSCHRIFT

(11) **EP 3 149 150 B1**
(45) Veröffentlichungstag und Bekanntmachung des Hinweises auf die Patenterteilung: **22.11.2017**
(21) Anmeldenummer: 15723473.3
(22) Anmeldetag: 11.05.2015
(51) Int. Cl.: C12M 1/34, C12M 1/36, G01N 35/00

(54) **AUTOMATISCHES VERFAHREN ZUR BEOBACHTUNG VON ZELLKULTURWACHSTUM**
AUTOMATIC METHOD FOR MONITORING CELL CULTURE GROWTH
PROCÉDÉ AUTOMATISÉ POUR L'OBSERVATION DE LA CROISSANCE D'UNE CULTURE CELLULAIRE

(30) Priorität: 07.10.2014 DE 102014220306
(43) Veröffentlichungstag der Anmeldung: 05.04.2017
(73) Patentinhaber: Fraunhofer-Gesellschaft zur Förderung der angewandten Forschung e.V., 80686 München (DE)
(72) Erfinder: DRÖGE, Marc, 59071 Hamm (DE); MÜLLER, Christian, 53783 Eitorf (DE); LINNEMANN, Anja, 53757 Sankt Augustin (DE); MATHIS, Harald Peter, 53175 Bonn (DE); BORBE, Stefan, 53757 Sankt Augustin (DE); HEUP, Torsten, 50129 Bergheim-Fliesteden (DE); PIPPOW, Andreas, 50968 Köln (DE)
(74) Vertreter: dompatent von Kreisler Selting Werner - Partnerschaft von Patent- und Rechtsanwälten mbB
(86) Internationale Anmeldenummer: PCT/EP2015/060297
(87) Internationale Veröffentlichungsnummer: WO 2016/055169

(56) Entgegenhaltungen:
- WO-A2-2010/121601
- WO-A2-2010/129532
- DE-A1-102006 007 197
- DE-A1-102012 223 128
- DE-B3-102012 022 603
- DE-T2- 69 409 649
- M. FREDBORG ET AL: "Real-Time Optical Antimicrobial Susceptibility Testing", JOURNAL OF CLINICAL MICROBIOLOGY, Bd. 51, Nr. 7, 17. April 2013 (2013-04-17) , Seiten 2047-2053, XP55157713, ISSN: 0095-1137, DOI: 10.1128/JCM.00440-13 in der Anmeldung erwähnt

## Beschreibung

Die Erfindung betrifft ein automatisches Verfahren zur insbesondere Echtzeitverfolgung von Zellkulturwachstum und insbesondere von Bakterienwachstum.

Es existieren verschiedene Anwendungsgebiete, bei denen es von Interesse ist, ein Zellkulturwachstum möglichst in Echtzeit beobachten zu können. Als ein Beispiel sei die Bestimmung von Antibiotika-Resistenz/Wirksamkeit genannt.

Das konventionelle Verfahren zur Bestimmung von Antibiotika-Resistenzen bzw. zur Bestimmung der Wirksamkeit von Antibiotika basiert auf einer makroskopischen Betrachtungsweise. Dieses Verfahren bedarf einer relativ großen Dauer, da Wachstumsbeurteilungen erst dann erfolgen können, wenn Bakterienkolonien mit dem bloßen Auge zu erkennen sind. Dies kann zwischen 6 bis 24 Stunden der Fall sein, was in zeitkritischen Fällen nicht tolerabel ist.

In WO-A-2010/129532 ist ein Verfahren und eine Vorrichtung für den schnellen Nachweis resistenter Bakterien beschrieben. Hierbei wird werden immobilisierte Bakterienzellen in einem Kulturmedium mit oder ohne Antibiotikum mittels eines Mikroskops beobachtet.

Des Weiteren ist in Marlene Fredborg et al., "Real-Time Optical Antimicrobial Susceptibility Testing", Journal of Clinical Microbiology, Band 51, Heft 7, 2013, S. 2047-2053, eine Vorrichtung zur Bildverarbeitung für Tests auf Antibiotikaresistenz beschrieben.

Unter der Bezeichnung VITEK® 2existiert ein alternatives, semiautomatisches Verfahren für die schnelle Empfindlichkeitstestung von Mikroorganismen. Hierbei wird eine Transmissionsoptik eingesetzt. Die Menge von eine Probe durchscheinendem Licht gibt dabei Rückschlüsse auf das Zellkulturwachstum. Je stärker das Wachstum ist, desto weniger intensiv ist das transmittierte Licht. Allerdings liefert auch dieses Verfahren zuverlässig erste Ergebnisse leider erst frühestens nach einigen Stunden.

Die beiden zuvor genannten Verfahren sind nicht in der Lage, die Wirkung von Antibiotika auf einzelne Zellen zu bestimmen, sondern erfordern eine Vielzahl von zu beobachtenden Zellen bzw. die Trübung einer Bakteriensuspension.

In DE-T-694 09 649 ist ein Verfahren zur Bestimmung von in Materialien vorhandenen Mikroorganismen und zur Vorhersage ihres Wachstums beschrieben. Bei dem Verfahren können Stoffe/Materialien/Nährmedien zur Unterstützung oder Hemmung des mikrobiellen Wachstums oder aufgrund ihrer mikrobiziden Wirkung ausgewählt werden. Arzneimittel wie beispielsweise Antibiotika müssen auf Wirkung gegen bestimmte Mikroorganismen durchgemustert und die zur Erzielung der Wirkung erforderliche Konzentration bestimmt werden.

WO-A-2010/121601 offenbart ein Verfahren zur automatisierten, parallelisierten Kultivierung von Zellen in Zellkulturgefäßen und zur kontinuierlichen Beobachtung des Zellwachstums mittels Kameramikroskop.

In DE-A-10 2012 223 123 ist ein Autofokusverfahren für Mikroskope mit einem Objektiv beschrieben, das eine in einer Objektebene liegende Probe abbildet.

In WO-A-2011/066837 wird ein weiteres Verfahren zur Bestimmung des Zellwachstums von Mikroorganismen beschrieben. Das Wachstum wird dabei durch statistische Verfahren abgeschätzt.

Aus US-A-2009/0185734 sind eine Messvorrichtung und ein Verfahren zur Analyse von Partikeln in einer Flüssigkeitsprobe bekannt. Dabei kann es sich bei den Partikeln um tierische oder menschliche Zellen handeln.

In der Veröffentlichung BURNHAM, D. [u.a.]: "Rapid Ertapenem Susceptibility Testing and Klebsiella pneumoniae Carbapenemase Phenotype Detection in Klebsiella pneumoniae Isolates by Use of Automated Microscopy of Immobilized Live Bacterial Cells", J. Clin. Microbiol. (2014/ 52 (3) 982-986 ist ein Verfahren zur Evaluierung einer Ertapenem-Resistenz von Bakterien unter Zuhilfenahme eines automatischen Mikroskopiesystems beschrieben.

EP-A-2 708 890 beschreibt ein Verfahren zur Auswahl einer monoklonalen Zellkolonie, wobei eine Vielzahl von Zellkulturräumen bereitgestellt wird, in denen eine Suspension mit Zellen auf ein Nährmedium aufgetragen wird.

Aus US-A-2007/0069106 ist ein Verfahren zur Erfassung eines fokussierten Bildes bekannt.

US-A-2012/0013727 offenbart, die Fokusebene einer Probe in Bezug zu einer Kamera mittels eines Schrittmotors zu verändern.

Ferner zeigt die Doktorarbeit von Schumann, E. "Wechselspiel zwischen Doppelstrangbruch-Reparatur und Zellzyklus-Kontrolle am G2/M-Übergang" ein mikroskopisches Untersuchungsverfahren für eine Zellprobe.

Schließlich zeigt US-A-2011/0017902 ein weiteres Verfahren zur Zielfokuspositionierung bei einer Aufnahmeeinrichtung mit Autofokus.

Aufgabe der Erfindung ist es, ein automatisiertes Verfahren zur Beobachtung von Zellkulturwachstum, insbesondere von Bakterienwachstum, anzugeben, bei dem die Wachstumsbeurteilung wesentlich schneller als bei bekannten Verfahren möglich ist.

Zur Lösung dieser Aufgabe wird mit der Erfindung ein automatisiertes Verfahren nach Anspruch 1 zur Beobachtung von Zellkulturwachstum, insbesondere von Bakterienwachstum vorgeschlagen. Einzelne Ausgestaltungen des Verfahrens sind Gegenstand der jeweiligen Unteransprüche.

Bei dem erfindungsgemäßen Verfahren wird eine Bildverarbeitungs-Software eingesetzt, die zur Auswertung von in zeitlichen Abständen automatisch aufgenommenen Bildern der Zellkultur eingesetzt wird. Ein Nährmedium mit der Zellkultur wird in eine insbesondere miniaturisierte Aufnahmeschale appliziert. Das Nährmedium enthält ein oder mehrere Reagenzien, insbesondere Antibiotika. Bei der Zellkultur handelt es sich insbesondere um Bakterien, die auf ihre Antibiotika-Resistenz oder anhand derer die Wirksam eines Antibiotikums getestet werden sollen.

Die in zeitlichen Abständen erfolgende automatische Bilderstellung geschieht mit Hilfe eines (insbesondere Auflicht- oder Durchlicht-)Mikroskops mit Kamera und einer Aufnahmeoptikeinheit sowie einem Träger für die Aufnahmeschale, die relativ zueinander längs der optischen Achse insbesondere in Kleinstschritten verfahrbar sind. Dies ermöglicht es, die Bildaufnahmeebene stets automatisch derart zu wählen, dass möglichst kontrastreiche Bilder von der Zellkultur aufgenommen werden können. Hierzu wird die Bildaufnahmeebene durch Verfahren der Aufnahmeoptikeinheit und/oder des Trägers längs der optischen Achse durch das Nährmedium hindurch bewegt, wobei in den unterschiedlichen Bildaufnahmeebenen jeweils ein Bild aufgenommen wird. Durch eine nachfolgende oder zeitgleich ablaufende Bildverarbeitung wird möglichst das kontrastreichste Bild ermittelt und ausgewählt. Alternativ reicht es möglicherweise aus, aus der Gruppe der jeweils aufgenommenen Bilder eines der kontrastreichsten auszuwählen, solange der Kontrast ausreichend groß ist, um die nachfolgend genannte Bildauswertung durchführen zu können. Derartige Bildverarbeitungen werden z.B. in Autofokus-Kameras eingesetzt und können dementsprechend bei der Erfindung genutzt werden.

Zwecks Auswählens des kontrastreichsten Bildes oder des Bildes mit für die nachfolgende Bildweiterverarbeitung ausreichendem Kontrast wird erfindungsgemäß das als erstes aufgenommene Bild als das bis dahin kontrastreichste Bild ermittelte Bild abgespeichert werden, um jedes weitere aufgenommene Bild mit dem abgespeicherten Bild zu vergleichen und, wenn es kontrastreicher ist, als das bis dahin kontrastreichste ermittelte Bild abzuspeichern.

Durch ein weiteres Bildverarbeitungs-Softwaremodul kann nun das jeweils ausgewählte Bild im Hinblick auf die Größe der von der Zellkultur eingenommenen Fläche automatisch untersucht werden. Damit lässt sich also quasi in Echtzeit das Wachstum der Zellkultur beobachten. Ein Beispiel einer hierfür geeigneten Software ist in Andreas Pippow, Stefan Borbe, Sebastian Röse, Stefan Precht, Thomas Berlage, "Zellteilungsdauer im High-Content-Screening bestimmen", Laborwelt, Nr. 1/2012, S. 33 und 34, und Thomas Berlage, Andreas Pippow, "Neues Potential für die Pharmaforschung", GIT Labor-Fachzeitschrift, 10/2013, S. 630 bis 635, beschrieben

Ein wesentliches Merkmal der Erfindung ist die Möglichkeit der automatischen Bildaufnahme bei möglichst optimalem Kontrast, wofür es der Verfahrbarkeit der Aufnahmeoptikeinheit und/oder des Trägers des Mikroskops wie bei einer Autofokus-Funktion bedarf. Die Bildaufnahmeebene muss zwecks Aufnahme von kontrastreichen Bildern allein deshalb schon von Zeit zu Zeit nachgeführt werden, weil das Nährmedium im Laufe der Zeit verdunsten könnte und somit sich der Nährmedium-Spiegel in der Aufnahmeschale verändert.

Das Mikroskop weist zweckmäßigerweise einen entlang der optischen Achse insbesondere in kleinen Stufen von wenigen zig µm verfahrbaren Träger für die Aufnahmeschale auf. Man könnte auch darüber nachdenken, die Aufnahmeoptikeinheit des Mikroskops längs der optischen Achse zu verfahren, was aber wesentlich aufwendiger ist als das Verfahren des Trägers. Hierbei bedarf es eines Antriebs, der ein Verfahren in zweckmäßigerweise Kleinstschritten erlaubt.

In weiterer zweckmäßiger Ausgestaltung der Erfindung kann vorgesehen sein, dass anhand der Größen der von der Zellkultur eingenommenen Flächen der jeweiligen ausgewählten Bilder ermittelt wird, ob die Zellkultur wächst und, wenn ja, mit welcher Wachstumsrate.

Wie bereits oben erwähnt, ist davon auszugehen, dass zu den jeweiligen Zeitpunkten, zu denen das nächste möglichst kontrastreiche Bild aufgenommen werden soll, sich auch die Bildaufnahmeebene gegenüber der jeweils früheren Position verändert hat. Aus Gründen der Zeiteinsparung kann es statt des jeweiligen zur Erzielung eines möglichst kontrastreichen Bildes erfolgenden Durchfahrens der Bildaufnahmeebene durch das Nährmedium von Vorteil sein, wenn der Bereich, innerhalb dessen die Bildaufnahmeebene in dem Nährmedium zur Ermittlung des Bildes des Nährmediums mit größtem Kontrast oder zur Ermittlung eines Bildes des Nährmediums mit einem für die Ermittlung der Größe der Fläche der Zellkultur ausreichendem Kontrast auf den Bereich um die Position der Bildaufnahmeebene des zeitlich letzten Bildes oder eines der zeitlich vorherigen Bilder beschränkt wird. Hierbei wird davon ausgegangen, dass sich die Bildaufnahmeebene, in der das vermeintlich schärfste Bild aufgenommen werden kann, von einem Aufnahmezeitpunkt zum nächsten Aufnahmezeitpunkt kaum verändert. Allerdings kann das jeweils vollständige Durchfahren der Bildaufnahmeebene durch das Nährmedium zu den einzelnen Bildaufnahmezeitpunkten deshalb erforderlich sein, weil die Autofokus-Einheit aufgrund äußerer Einflüsse, beispielsweise thermischer Einflüsse oder Vibrationen, seit dem letzten Bildaufnahmezeitpunkt verjustiert ist.

Wie bereits oben erwähnt, ist es aus verschiedenen Gründen zweckmäßig, eine möglichst kleinformatige Aufnahmeschale zu verwenden. Eine Möglichkeit ist es, eine miniaturisierte Aufnahmeschale zu verwenden, die zwei Folien mit einem zwischen diesen angeordneten Rahmen aufweist. Bei den beiden Folien handelt es sich beispielsweise um Objektträgergläser. Der Rahmen umgibt dabei einen Aufnahmeraum, der durch den Rahmen selbst seitlich und durch die beiden Folien nach oben und nach unten geschlossen ist. In diesen Aufnahmeraum wird dann das Nährmedium (z.B. Agar) appliziert, welches mit den zu beobachtenden Zellkulturen und einem oder mehreren Reagenzien versetzt ist.

Wie sich aus dem Obigen ergibt, ist also Gegenstand der vorliegenden Erfindung ein automatisiertes Verfahren zur Echtzeitverfolgung von Bakterienwachstum bzw. von Zellkulturwachstum bzw. von Wachstum von Mikroorganismen. Das Verfahren basiert auf der Verwendung eines Mikroskops, welches in definierten Zeitabständen, unterstützt durch eine Autofokus-Einheit, Aufnahmen von mit den Zellkulturen bewachsenen, insbesondere miniaturisierten Wachstumsflächen (Nährmedium) erstellt. Die Aufnahmen bzw. Bilder werden anschließend von einer Bildverarbeitungs-Software vorverarbeitet und analysiert, um letztendlich anhand der Größe der Fläche der Bilder, die zu den jeweiligen Zeitpunkten aufgenommen und ausgewertet worden sind, eine Wachstumskurve oder eine andere Art der Repräsentation des Wachstums der Zellkultur zu erstellen.

Bei dem erfindungsgemäß automatisierten Verfahren zur Beobachtung von Zellkulturwachstum, insbesondere von Bakterienwachstum wird also eine Aufnahmeschale mit einem Nährmedium bereitgestellt, auf und/oder in das eine Zellkultur, insbesondere eine mit Bakterien versetzte Probe menschlichen oder tierischen Gewebes wie z.B. Blut, welche zusätzlich ein oder mehrere Reagenzien, insbesondere Antibiotika enthält, appliziert ist. Ein Mikroskop mit Kamera und entlang der optischen Achse verfahrbaren Bildaufnahmeebene wird bereitgestellt und die Aufnahmeschale zur Beobachtung eines potentiellen Wachstums der Zellkultur wird in dem Nährmedium in das Mikroskop verbracht. In vorgebbaren zeitlichen Abständen im insbesondere einstelligen Minutenbereich wird mittels der Kamera des Mikroskops ein Bild des Nährmediums aufgenommen wird, indem Bildaufnahmeebene entlang der optischen Achse durch die Aufnahmeschale das Nährmedium hindurch verfahren wird, pro Bildaufnahmeebene ein Bild aufgenommen wird und mittels einer Bildauswerte-Software aus der Gruppe der aufgenommenen Bilder das kontrastreichste Bild des Nährmediums oder ein Bild des Nährmediums mit für die nachfolgend genannte automatische Bildweiterverarbeitung ausreichendem Kontrast automatisch ausgewählt und gegebenenfalls gespeichert wird. Mittels der Bildauswerte-Software wird anhand des ausgewählten Bildes automatisch die Größe der von der Zellkultur eingenommenen Fläche ermittelt. Anhand der Größen der von der Zellkultur eingenommenen Flächen der jeweiligen ausgewählten Bilder wird ermittelt, ob die Zellkultur wächst oder nicht.

Das erfindungsgemäße Verfahren ermöglicht die Echtzeitverfolgung von Bakterienwachstum auf der Ebene von Bakterienkolonien sowie auf zellulärer Ebene, was bedeutet, dass einerseits durch die Beobachtung einer Wachstumsflächenzunahme schnell eine Wachstumskurve abgeleitet werden kann und andererseits zusätzlich die Zellteilung jedes einzelnen Individuums verfolgt werden kann. Dies ermöglicht eine Beschleunigung gegenüber konventionellen Resistenztests, die Bereitstellung eines neuen Ansatzes für die beispielsweise Antibiotika-Forschung sowie die automatisierte Verfolgung von Bakterienwachstum/Bakterienteilung über kurze und lange Zeiträume.

Die Wirkungen und Vorteile der Erfindung lassen sich wie folgt zusammenfassen:
- Durch den Einsatz von optimierten, miniaturisierten Wachstumsflächen wird eine automatisierte, mikroskopische Verfolgung des Wachstums auf zellulärer Ebene ermöglichst. Zusätzlich werden durch die Miniaturisierung der Wachstumsfläche Reagenzien und Probematerial eingespart, was einen wirtschaftlichen Vorteil gegenüber der konventionellen Methode mit sich bringt.
- Durch die Verwendung einer Autofokus-Einheit des Mikroskops wird eine automatisierte Erstellung von Aufnahmen/Bildern in bestimmten Zeitabständen möglich. Ohne die Autofokus-Einheit wäre ein manuelles Nachfokussieren vor jeder neuen Aufnahme nötig, was eine Automatisierung zuwiderlaufen würde.
- Durch die Erstellung von Wachstumskurven aus der von beispielsweise Bakterien bewachsenen Fläche kann die Bestimmung der minimalen Hemmkonzentration (MHK) von Antibiotika signifikant, nämlich in 2 bis 3 Stunden statt, wie konventionell, in 5 bis 24 Stunden, beschleunigt werden.

Neben der Erstellung einer Wachstumskurve anhand einer auf Vorder-/Hintergrunderkennung basierten Flächenbestimmung basiert die Erfindung ferner auf der Verwendung einer auf Kontraststärke ausgerichteten Autofokus-Funktion und der Verwendung insbesondere miniaturisierter Wachstumsflächen (beispielsweise auf Agarbasis oder einer anderen Nährmediumbasis).

Anwendungsfälle für das erfindungsgemäße Verfahren sind die
- Beobachtung von (bakteriellen) Zellteilungen,
- Bestimmung von Antibiotika-Resistenzen,
- Beobachtung des Einflusses diverser Stoffe auf das Bakterienwachstum bzw. die Zellteilungen und
- Beobachtung des Einflusses kombinatorischer Antibiotika-Gabe auf das Bakterienwachstum bzw. die Zellteilungen.

Bildverarbeitungs-Software zur automatischen Ermittlung der Größe von ausgewählten Bereichen eines Bildes wie im vorliegenden Fall der von der Zellkultur eingenommenen Fläche, ist grundsätzlich bekannt. Ein Beispiel ist die unter der Bezeichnung ZETA von der Anmelderin entwickelte Software (siehe auch die beiden oben angegebenen Nichtpatentliteraturstellen).

Die Erfindung wird nachfolgend anhand der Zeichnung näher erläutert. Im Einzelnen zeigen dabei:
- Fig. 1: schematisch eine Darstellung eines erfindungsgemäß verwendbaren Auflicht-Mikroskops,
- Fig. 2: eine Vergrößerung der Objektträgeranordnung mit miniaturisierter Aufnahmeschale,
- Fig. 3: schematisch das Funktionsprinzip zur automatischen Ermittlung des kontrastreichsten Bildes,
- Fig. 4: Beispiele für Aufnahmen der wachsenden Zellkultur zu verschiedenen Zeitpunkten A, B und C und die diesen Aufnahmen entsprechenden, aufgearbeiteten Bilder zur Vordergrund/Hintergrunderkennung zwecks automatischer Zellkultur-Flächenermittlung und
- Fig. 5: drei Beispiele für erfindungsgemäß erstellte Wachstumskurven.

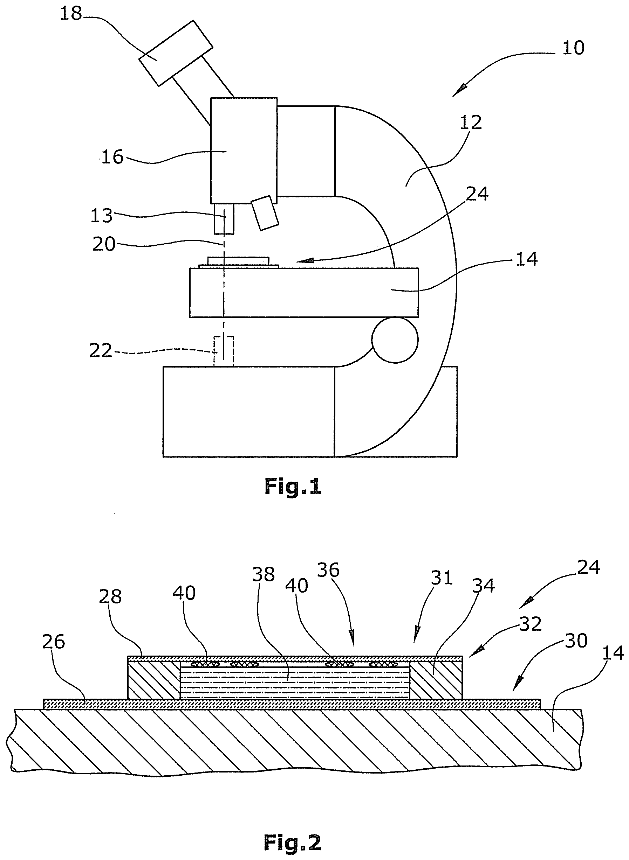

In Fig. 1 ist ein in diesem Ausführungsbeispiel Auflicht-Mikroskop 10 gezeigt, das einen Mikroskopkörper 12 mit Auflicht-Beleuchtungseinheit 13 und einem insbesondere in Kleinstschritten längs der optischen Achse des Mikroskops verfahrbaren Träger 14 (verfahrbarer Z-Tisch) aufweist. Der Mikroskopkörper 12 umfasst ferner eine Aufnahmeoptikeinheit 16 und eine Kamera 18. Die optische Achse ist bei 20 angedeutet.

Alternativ zu dem Auflicht-Mikroskop kann nach der Erfindung auch ein Durchlicht-Mikroskop eingesetzt werden. Die Positionierung der in diesem Fall einzusetzenden Durchlicht-Beleuchtungseinheit ist gestrichelt bei 22 gezeigt.

Auf dem Träger 14 befindet sich eine Aufnahmeschale 24, die in Fig. 2 in größerem Maßstab gezeigt ist. Die Aufnahmeschale 24 weist einen unteren Objektträger 26 und ein Deckglas 28 als insbesondere zwei Folien oder Scheiben 30,31 auf, zwischen denen ein insbesondere als doppelseitiger Klebefilm 32 ausgebildeter Rahmen 34 angeordnet ist. Dieser Rahmen 34 definiert in seinem Innern den Aufnahmeraum 36 für ein Nährmedium 38 (bei dem es sich beispielsweise um Agar handelt), auf dem Bakterien 40 wachsen. Das Nährmedium ist mit einem Reagenz, wie beispielsweise einem Antibiotikum versehen. Die Bakterien 40 können Teil beispielsweise einer Blutprobe, die es zu untersuchen gilt, sein.

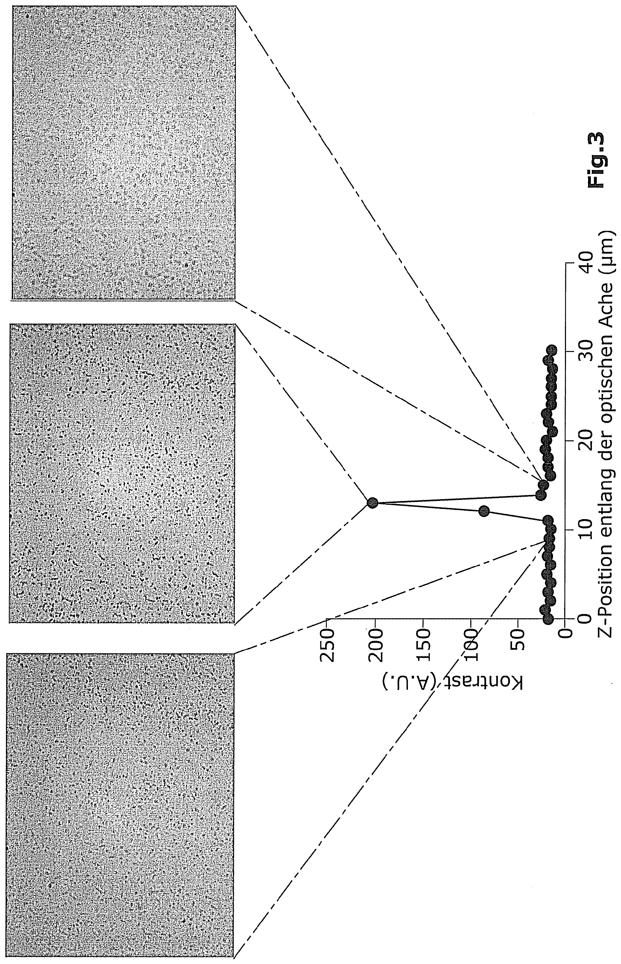

In Fig. 3 ist das Funktionsprinzip der automatischen Ermittlung des kontrastreichsten Bildes, welches zu einem bestimmten Zeitpunkt nach Beginn des Bakterienwachstums aufgenommen wird, dargestellt. Mittels des in Kleinstschritten verfahrbaren Trägers 14 wird die Bildaufnahmeebene durch die Aufnahmeschale 24 hindurch verfahren. Dabei wird pro Schritt ein Bild aufgenommen. Abgefragt wird dabei die Kontraststärke in verschiedenen Z-Positionen. In Fig. 3 ist dies am Beispiel der Aufnahme einer Bakterienkultur aus *Staphylococcus aureus* gezeigt. Das Diagramm der Fig. 3 zeigt die Kontraststärke (in A.U.) in Abhängigkeit von der Z-Position (in µm). Beispielhaft sind drei Bilder mit den ihnen zugeordneten Kontraststärken gezeigt.

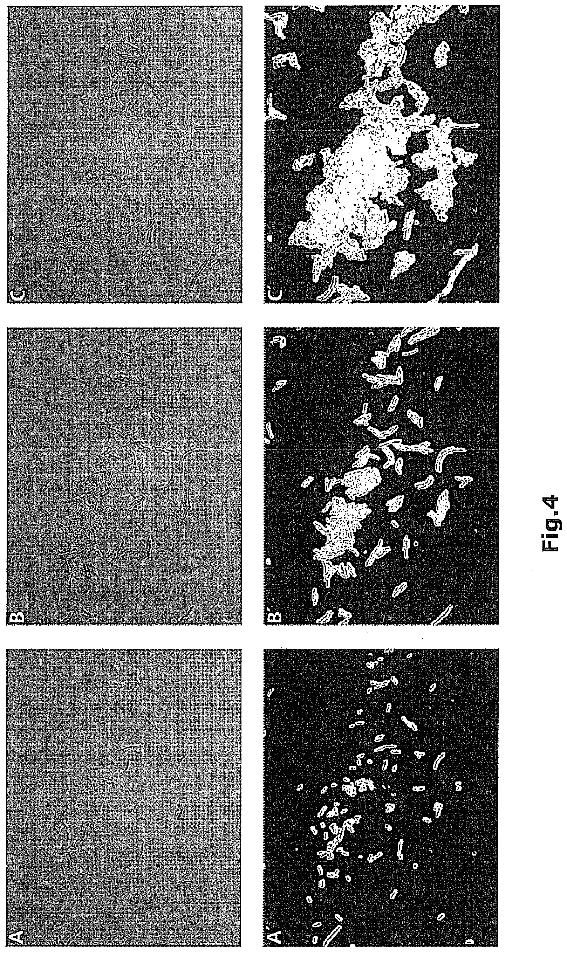

Fig. 4 zeigt Beispiele für die Vordergrund/Hintergrunderkennung durch die bereits oben erwähnte ZETA-Software der Anmelderin am Beispiel einer *Escherichia Coli (E.coli)*-Wachstumsanalyse. Die obere Reihe (A-C) zeigt die Originalbilder, wobei A den Anfang einer Zeitreihe darstellt, B nach etwa einer Stunde und C nach 6 Stunden aufgenommen ist. Die zweite, untere Reihe (A'-C') zeigt die entsprechenden Vordergrund/Hintergrunderkennungen als Schwarz-Weiß-Darstellungen.

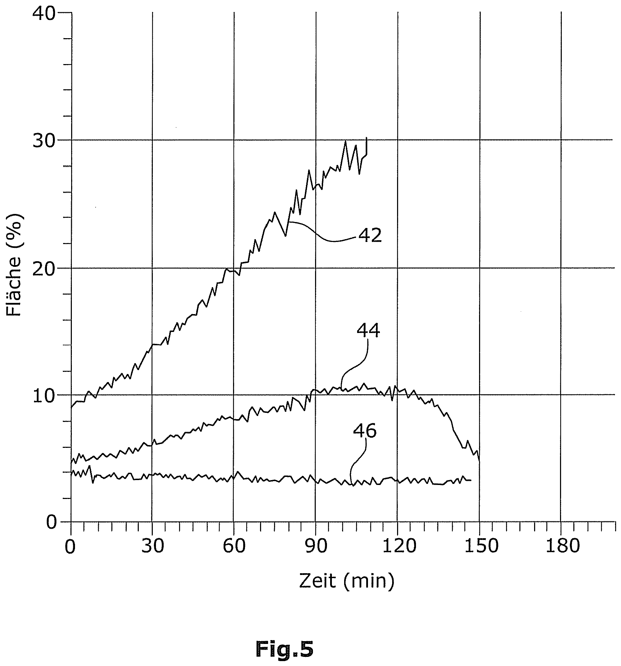

Fig. 5 schließlich zeigt drei Beispiele für erfindungsgemäß ermittelte Bakterienwachstumskurven eines antibiotischen Resistenztests *E.coli* für LB-Medium (siehe Kurve 42), für Ampicillin (siehe Kurve 44) und für Kanamycin (siehe Kurve 46).

## Patentansprüche

1. Automatisches Verfahren zur Beobachtung von Zellkulturwachstum, insbesondere von Bakterienwachstum, wobei bei dem Verfahren
- eine Aufnahmeschale (24) mit einem Nährmedium (38) bereitgestellt wird, auf dem eine Zellkultur, insbesondere eine mit Bakterien versetzte Probe menschlichen oder tierischen Gewebes wie z.B. Blut, welche zusätzlich ein oder mehrere Reagenzien, insbesondere Antibiotika enthält, appliziert ist,
- ein eine optische Achse (20) aufweisendes Mikroskop (10) mit Kamera (18) und einem längs der optischen Achse (20) automatisch verfahrbaren Träger (14) für die Aufnahmeschale (24) und/oder einer längs der optischen Achse (20) automatisch verfahrbaren Aufnahmeoptikeinheit (16) bereitgestellt wird,
- die Aufnahmeschale (24) zur Beobachtung eines potentiellen Wachstum der Zellkultur in dem Nährmedium (38) in das Mikroskop (10) verbracht wird,
- in vorgebbaren zeitlichen Abständen im insbesondere einstelligen Minutenbereich mittels der Kamera (18) des Mikroskops (10) ein Bild des Nährmediums (38) aufgenommen wird, indem mittels des verfahrbaren Trägers (14) und/oder der Aufnahmeoptikeinheit (16) des Mikroskops (10) die Bildaufnahmeebene entlang der optischen Achse (20) durch das Nährmedium (38) hindurch verfahren wird, pro Bildaufnahmeebene ein Bild aufgenommen wird und mittels einer Bildauswerte-Software aus der Gruppe der aufgenommenen Bilder das kontrastreichste Bild des Nährmediums (38) oder ein Bild des Nährmediums (38) mit für die nachfolgend genannte automatische Bildweiterverarbeitung ausreichendem Kontrast automatisch ausgewählt und gegebenenfalls gespeichert wird,
- wobei zum Auswählen des kontrastreichsten Bildes oder zum Auswählen des Bildes mit für die nachfolgende Bildweiterverarbeitung ausreichendem Kontrast das als erstes aufgenommene Bild als das bis dahin kontrastreichste ermittelte Bild abgespeichert wird und jedes weitere aufgenommene Bild mit dem abgespeicherten Bild verglichen wird und, wenn es kontrastreicher ist als das abgespeicherte Bild, als das bis dahin kontrastreichste ermittelte Bild abgespeichert wird,
- mittels der Bildauswerte-Software anhand des ausgewählten Bildes automatisch die Größe der von der Zellkultur eingenommenen Fläche ermittelt wird und
- anhand der Größen der von der Zellkultur eingenommenen Flächen der jeweiligen ausgewählten Bilder ermittelt wird, ob die Zellkultur wächst oder nicht.

2. Verfahren nach Anspruch 1, **dadurch gekennzeichnet, dass** anhand der Größen der von der Zellkultur eingenommenen Flächen der jeweiligen ausgewählten Bilder ermittelt wird, ob die Zellkultur wächst und, wenn ja, mit welcher Wachstumsrate.

3. Verfahren nach Anspruch 1 oder 2, **dadurch gekennzeichnet, dass** der Schritt des Durchfahrens der Bildaufnahmeebene durch die Aufnahmeschale (24) und das Nährmedium (38) durch Verfahren von optischen Elementen der Aufnahmeoptikeinheit (16) des Mikroskops (10) längs der optischen Achse (20) und/oder durch Verfahren des die Aufnahmeschale (24) aufweisenden Trägers (14) längs der optischen Achse (20) des Mikroskops (10) erfolgt.

4. Verfahren nach einem der Ansprüche 1 bis 3, **dadurch gekennzeichnet, dass** der Bereich, innerhalb dessen die Bildaufnahmeebene in dem Nährmedium (38) zur Ermittlung des Bildes des Nährmediums (38) mit größtem Kontrast oder zur Ermittlung eines Bildes des Nährmediums (38) mit einem für die Ermittlung der Größe der Fläche der Zellkultur ausreichendem Kontrast auf den Bereich um die Position der Bildaufnahmeebene des zeitlich letzten Bildes oder eines der zeitlich vorherigen Bilder beschränkt wird.

5. Verfahren nach einem der Ansprüche 1 bis 4, **dadurch gekennzeichnet, dass** die Aufnahmeschale (24) zwei Folien, insbesondere Objektträger (26) und Deckglas (28), mit einem zwischen diesen angeordneten Rahmen (34) aufweist, wobei der von dem Rahmen (34) umgebene Bereich den Aufnahmeraum (36) der Aufnahmeschale (24) bildet.

6. Verfahren nach einem der Ansprüche 1 bis 5, **dadurch gekennzeichnet, dass** anhand der Größen der von der Zellkultur eingenommenen Flächen der jeweiligen ausgewählten Bilder eine Wachstumskurve erstellt wird.

7. Verfahren nach einem der Ansprüche 1 bis 6, **dadurch gekennzeichnet, dass** als Mikroskop (10) ein Auflicht- oder Durchlichtmikroskop ausgewählt wird.

## Claims

1. An automated method for monitoring cell culture growth, in particular bacterial growth, wherein in the method
- a receiving dish (24) with a nutrient medium (38) is provided onto which is applied a cell culture, in particular a sample of human or animal tissue, such as e.g. blood, to which bacteria have been added and which also contains one or a plurality of reagents, in particular antibiotics,
- a microscope (10) having an optical axis (20) and including a camera (18) and a slide (14) for the receiving dish (24) adapted to be automatically moved along the optical axis (20) and/or an acquisition optic unit (16) adapted to be automatically moved along the optical axis (20) is provided,
- the receiving dish (24) is brought into the microscope (10) for monitoring a potential growth of the cell culture in the nutrient medium (38),
- at predetermined time intervals, in particular in the single-digit minute range, an image of the nutrient medium (38) is acquired using the camera (18) of the microscope (10) by moving the image acquisition plane along the optical axis (20) through the nutrient medium (38) by means of at least one of the movable slide (14) and/or the acquisition optic unit (16) of the microscope (10), one image per image acquisition plane is acquired, and, from the group of acquired images, the highest-contrast image of the nutrient medium (38) or an image of the nutrient medium (38) with a contrast sufficient for the subsequent automatic further processing of the image is automatically selected and stored, where required, by means of an image evaluation software,
- for selecting the highest-contrast image or for selecting the image with a contrast sufficient for the subsequent further processing of the image, the image acquired first is stored as the image determined as having the highest contrast so far, and each further acquired image is compared to the stored image, and if this image has a higher contrast than the stored image, it is stored as the image determined as having the highest contrast so far,
- by means of the image evaluation software, the size of the area occupied by the cell culture is automatically determined on the basis of the selected image, and
- on the basis of the sizes of those areas of the respective selected images that are occupied by the cell culture, it is determined whether the cell culture is growing or not.

2. The method according to claim 1, **characterized in that**, on the basis of the sizes of said areas of the respective selected images that are occupied by the cell culture, it is determined whether the cell culture is growing, and if so, at which growth rate.

3. The method according to claim 1 or 2, **characterized in that** the step of the image acquisition plane traveling through the receiving dish (24) and through the nutrient medium (38) is effected by moving optical elements of the acquisition optic unit (16) of the microscope (10) along the optical axis (20) and/or by moving the slide (14) comprising the receiving dish (24) along the optical axis (20) of the microscope (10).

4. The method according to any one of claims 1 to 3, **characterized in that** the region within which the image acquisition plane travels in the nutrient medium (38) for determining the image of the nutrient medium (38) with the highest contrast or for determining an image of the nutrient medium (38) with a contrast sufficient for determining the size of the area of the cell culture is limited to the region around the position of the image acquisition plane of the temporally last image or one of the temporally previous images.

5. The method according to any one of claims 1 to 4, **characterized in that** the receiving dish (24) comprises two sheets, in particular an object slide (26) and a cover slip (28) having a frame (34) arranged therebetween, wherein the region surrounded by the frame (34) defines the receiving space (36) of the receiving dish (24).

6. The method according to any one of claims 1 to 5, **characterized in that** on the basis of the sizes of said areas of the respective selected images that are occupied by the cell culture, a growth curve is prepared.

7. The method according to any one of claims 1 to 6, **characterized in that**, as the microscope (10), a reflected-light or a transmitted-light microscope is selected.

## Revendications

1. Procédé automatique destiné à l'observation de la croissance d'une culture cellulaire, en particulier de la croissance de bactéries, selon lequel, pour le procédé :
- une cuvette de réception (24) est préparée avec un milieu de culture (38), sur lequel est appliquée une culture cellulaire, en particulier un échantillon de tissu humain ou animal, tel que du sang par exemple, auquel des bactéries sont ajoutées et lequel contient en outre un ou plusieurs réactifs, en particulier des antibiotiques ;
- un microscope (10) présentant un axe optique (20) est préparé, avec une caméra (18), ainsi qu'avec un support (14) pour la cuvette de réception (24), lequel peut être déplacé automatiquement le long de l'axe optique (20), et/ou avec une unité optique d'acquisition d'images (16) qui peut être déplacée automatiquement le long de l'axe optique (20) ;
- la cuvette de réception (24) destinée à l'observation d'une croissance potentielle de la culture cellulaire dans le milieu de culture (38) est introduite dans le microscope (10) ;
- une image du milieu de culture (38) est réalisée au moyen de la caméra (18) du microscope (10) à des intervalles pouvant être prédéfinis dans le temps, en particulier dans une plage de minutes à un chiffre, dans la mesure où le plan d'acquisition d'images est déplacé le long de l'axe optique (20), à travers le milieu de culture (38), au moyen du support (14) pouvant être déplacé et/ou de l'unité optique d'acquisition d'images (16) du microscope (10) ;
selon lequel une image est réalisée par plan d'acquisition d'images ; et
selon lequel l'image plus riche en contraste du milieu de culture (38) ou une image du milieu de culture (38), laquelle présente un contraste suffisant pour le traitement automatique ultérieur mentionné ci-dessous, est automatiquement sélectionnée et, le cas échéant, enregistrée au moyen d'un logiciel d'évaluation des images à partir du groupe des images réalisées ;
- selon lequel l'image réalisée en premier est mémorisée comme étant l'image la plus riche en contraste acquise jusqu'à présent et chaque nouvelle image réalisée est comparée à l'image mémorisée, en vue de la sélection de l'image la plus riche en contraste ou en vue de la sélection de l'image qui présente un contraste suffisant pour le traitement automatique ultérieur mentionné ci-dessous, et si cette image est encore plus riche en contraste que l'image mémorisée, elle devient alors mémorisée comme étant l'image la plus riche en contraste acquise jusqu'à présent ;
- la taille de la surface occupée par la culture cellulaire est déterminée automatiquement au moyen du logiciel d'évaluation des images, à l'aide de l'image sélectionnée ; et
- il est déterminé, à l'aide des tailles des surfaces des images respectives sélectionnées qui sont occupées par la culture cellulaire, si la culture cellulaire est en croissance ou non.

2. Procédé selon la revendication 1, **caractérisé en ce qu'**il est déterminé, à l'aide des tailles des surfaces des images respectives sélectionnées qui sont occupées par la culture cellulaire, si la culture cellulaire est en croissance et, dans l'affirmative, selon quel taux de croissance.

3. Procédé selon la revendication 1 ou 2, **caractérisé en ce que** la phase du passage du plan d'acquisition d'images à travers la cuvette de réception (24) et le milieu de culture (38) a lieu par l'intermédiaire du déplacement d'éléments optiques de l'unité optique d'acquisition d'images (16) du microscope (10) le long de l'axe optique (20) et/ou par l'intermédiaire du déplacement du support (14) présentant la cuvette de réception (24) le long de l'axe optique (20) du microscope (10).

4. Procédé selon l'une des revendications 1 à 3, **caractérisé en ce que** la zone, à l'intérieur de laquelle est défini le plan d'acquisition d'images dans le milieu de culture (38) en vue de la détermination de l'image du milieu de culture (38) qui présente le plus grand contraste ou en vue de la détermination d'une image du milieu de culture (38) qui présente un contraste suffisant pour la détermination de la taille de la surface de la culture cellulaire, est limitée à la zone qui se trouve autour de la position du plan d'acquisition d'images de la dernière image obtenue chronologiquement, ou de l'une des dernières images précédemment obtenues.

5. Procédé selon l'une des revendications 1 à 4, **caractérisé en ce que** la cuvette de réception (24) présente deux films, en particulier une lame de microscope (26) et une lamelle couvre-objet (28), avec un cadre (34) disposé entre celles-ci, selon lequel la zone entourée par le cadre (34) forme la zone de capture d'images (36) de la cuvette de réception (24).

6. Procédé selon l'une des revendications 1 à 5, **caractérisé en ce qu'**une courbe de croissance est établie à l'aide des tailles des surfaces des images respectives sélectionnées qui sont occupées par la culture cellulaire.

7. Procédé selon l'une des revendications 1 à 6, **caractérisé en ce qu'**un microscope à lumière réfléchie ou un microscope à lumière transmise est sélectionné pour servir de microscope (10).
